# EUROPEAN PATENT APPLICATION

(11) **EP 1 283 058 A1**
(43) Date of publication of application: **12.02.2003**
(21) Application number: 01930173.8
(22) Date of filing: 15.05.2001
(51) Int. Cl.: A61K 45/00, A61P 43/00, A61P 3/10, A61K 31/519

(54) **AGENTS FOR PREVENTING OR AMELIORATING INSULIN RESISTANCE AND/OR OBESITY**

(30) Priority: 16.05.2000 JP 2000014349
(71) Applicant: Sanwa Kagaku Kenkyusho Co., Ltd., Nagoya-shi, Aichi 461-8631 (JP)
(72) Inventor: MIYAWAKI, Kazumasa, Kyoto-shi, Kyoto 606-8332 (JP); YAMADA, Yuichiro, Hirakata-shi, Osaka 573-1103 (JP); BAN, Nobuhiro, Kyoto-shi, Kyoto 606-0801 (JP); SEINO, Yutaka, Amagasaki-shi, Osaka 661-0003 (JP); TUBAMOTO, Yoshiharu SANWA KAGAKU KENKYUSHO CO. LTD, Nagoya-shi, Aichi 461-8631 (JP); TAKEDA, Motohiro SANWA KAGAKU KENKYUSHO CO. LTD, Nagoya-shi, Aichi 461-8631 (JP); HASHIMOTO, Hiroyuki SANWA KAGAKU KENKYUSHO CO. LTD, Nagoya-shi, Aichi 461-8631 (JP); YAMASHITA, Tokuyuki SANWA KAGAKU KENKYUSHO CO.LTD, Nagoya-shi, Aichi 461-8631 (JP); JOMORI, Takahito SANWA KAGAKU KENKYUSHO CO. LTD, Nagoya-shi, Aichi 461-8631 (JP)
(74) Representative: Lippert, Hans, Dipl.-Ing.
(86) International application number: JP0104058
(87) International publication number: WO01087341

(57) **Abstract**

An object of the present invention is to provide a prophylactic or ameliorative agent for insulin resistance and/or obesity based on a new concept, and a screening method therefor.

The present inventors found that GIP causes insulin resistance and obesity by a new mechanism, and obtained a new concept that a GIP function inhibitory compound exhibits the ameliorative effect of insulin resistance and anti-obesity effect, thereby completing the present invention. That is, the present invention is a prophylactic or ameliorative agent for insulin resistance and/or obesity, having a GIP function inhibitory compound as an active ingredient, and a screening method for a prophylactic or ameliorative agent for insulin resistance and/or obesity, characterized by selecting a GIP function inhibitory compound.

## Description

### TECHNICAL FIELD

The present invention relates to a prophylactic or ameliorative agent for insulin resistance and/or obesity, and more specifically to a prophylactic or ameliorative agent for insulin resistance and/or obesity , on the basis of a technological concept that a compound inhibiting a function of GIP (gastric inhibitory polypeptide or glucose-dependent insulinotropic peptide) serves as a prophylactic or ameliorative agent for insulin resistance and/or obesity, and a screening method therefor.

### BACKGROUND ART

GIP is one of gastrointestinal hormones belonging to Glucagon-secretin family. GIP, alongwith glucagon-like peptide 1 (GLP-1), is called incretin, is secreted on feeding from the K cells existing in the small intestine, and regulates the kinetics of nutrients after eating in the body by stimulating an insulin secretion on β-cells of the pancreas in response to glucose. In addition, GIP has been said to inhibit the gastric motor activity and stimulates the secretion of intestinal juice. However, a doubt exists at present as to its inhibitory effect on gastric acid secretion proposed just after its discovery. The GIP-receptor genes are expressed widely in addition to the β-cells in the pancreas and the adipocytes, so it is assumed that GIP has some effects in other tissues though they have not been clear in details. Naturally, its relationship with insulin resistance is unknown.

Examples of GIP-receptor antagonists include GIP(6-30)-NH₂ (Regulatory Peptide, 69: 151-154, 1997) and GIP(7-30)-NH₂ (Am J Physiol, 276: E1049-1054, 1999). However, they have not been studied as insulin resistance ameliorative agents or anti-obesity agents.

Insulin resistance represents a state where the glucose absorption promoting effect of insulin, that is the main effect of it, is weakened in the skeletal muscle, adipose tissue, and liver. If one has insulin resistance, the hypoglycemic effect is reduced even with the same amount of insulin existing in the body, and more amount of insulin is required in order to maintain the normal blood glucose level. This insulin resistance is largely involved in most Japanese patients with type 2 diabetes, and develops diabetes mellitus in the following course. In the early phase, a large amount of insulin is secreted from Langerhans islets in order to supplement insulin resistance, which maintains the normal blood glucose level but induce a condition of hyperinsulinemia. The function of Langerhans islets soon becomes too reduced to continue the secretion of a large amount of insulin, and it becomes impossible to supplement insulin resistance, leading to the increased blood glucose level. Pioglitazone (Actos) is the only prophylactic or ameliorative agent for insulin resistance that has been clinically used in Japan. Moreover, it is absolutely unknown that an inhibitor of GIP has prophylactic or ameliorative effect for insulin resistance.

On the other hand, obesity is a life style-related disease that has been more commonly observed in Japanese as our diet style has been westernized, and is a risk factor for such life style-related disease such as fattyliver, diabetes mellitus, gout, hypertension and arteriosclerosis. Medically, obesity is regarded as a pathological condition with abnormal fat accumulation as a result of relatively excessive calorie intake due to genetic and environmental conditions, and is a target of medical treatment. Treatment of obesity is usually provided through a combination of diet therapy and exercise therapy, and anorectics are rarely used. Mazindol (Sanorex) is the only drug currently used as a prophylactic or ameliorative agent for obesity in Japan, and studies on β-3 adrenoreceptor agonists, central active substance, digestion/absorption inhibitors, adipogenesis inhibitors and leptin are ongoing.

The fact that GIP antagonists have anti-obesity effect is described inWO98/24464. However, this cited reference only reports that GIP (7-30)-NH₂ is a GIP-receptor inhibitor and that it suppressed glucose absorption by the intestine. This cited reference does not document the effect of GIP(7-30) -NH₂ as a substantial anti-obesity agent, so that it is a logical leap to regard GIP(7-30)-NH₂ as an anti-obesity agent.

Other reports have showed that GIP promotes uptake of free fatty acid and glucose at the adipocyte level, though none of them clearly demonstrates its relationship with obesity.

An object of the present invention is to provide a prophylactic or ameliorative agent for insulin resistance and/or obesity based on a new concept, and a screening method therefor.

### DISCLOSURE OF THE INVENTION

The present inventors found in the course of studying the function of GIP that continuous administration of GIP to normal mice under high fat diet condition did not affect body weight gain but caused increases in fasting blood glucose levels and fasting plasma insulin levels depending on the dose of GIP. In other words, the inventors found that GIP exacerbated insulin resistance independently of body weight gain. It was also found in a high fat diet loading test with GIP-receptor gene-deficient mice that insulin resistance which was caused in wild type mice was ameliorated in GIP-receptor gene-deficient mice in terms of fasting blood glucose levels, insulin sensitivity and glucose tolerance. Moreover, inob/obmice, genetic obese mice, the defect of GIP-receptor gene also caused amelioration in the fasting blood glucose levels and glucose tolerance, showing insulin resistance ameliorative effect.

Based on these results, a new finding has been obtained that GIP causes insulin resistance by a novel mechanism that has so far not been suggested, and a new concept has been obtained that a compound inhibiting a function of GIP, such as a GIP-receptor antagonist or a GIP production inhibitor, has a prophylactic or ameliorative effect for insulin resistance.

On the other hand, the present inventors conducted a high fat diet loading test with GIP-receptor gene-deficient mice in the course of the study on the function of GIP, and found that obesity, which was developed in wild type mice, was suppressed in GIP-receptor gene-deficient mice. Moreover, in ob/ob mice, genetic obese mice, the defect of GIP-receptor gene could also inhibit obesity. Insulin resistance was also ameliorated in these systems.

Based on these results, a new finding has been obtained that GIP causes obesity by a novel mechanism that has so far not been suggested, and a new concept has been obtained that a compound inhibiting a function of GIP, such as a GIP-receptor antagonist or a GIP production inhibitor, has an anti-obesity effect, or has an insulin resistance ameliorative effect in association with an anti-obesity effect.

That is, the present invention is a prophylactic or ameliorative agent for insulin resistance and/or obesity, having a GIP function inhibitory compound as an active ingredient, and a screening method for a prophylactic or ameliorative agent for insulin resistance and/or obesity, characterized by selecting a GIP function inhibitory compound.

Such pharmacological effects of GIP were different from the conventionally known effects of GIP, which promoted insulin secretion or inhibited gastric acid secretion, and were not predicted easily. The present invention provides a completely novel concept that a compound inhibiting GIP function serves as a prophylactic or ameliorative agent for insulin resistance and/or obesity, and make it possible to obtain the prophylactic or ameliorative agent for insulin resistance and/or obesity by screening the GIP function inhibitory compound.

### BRIEF DESCRIPTION OF THE DRAWINGS

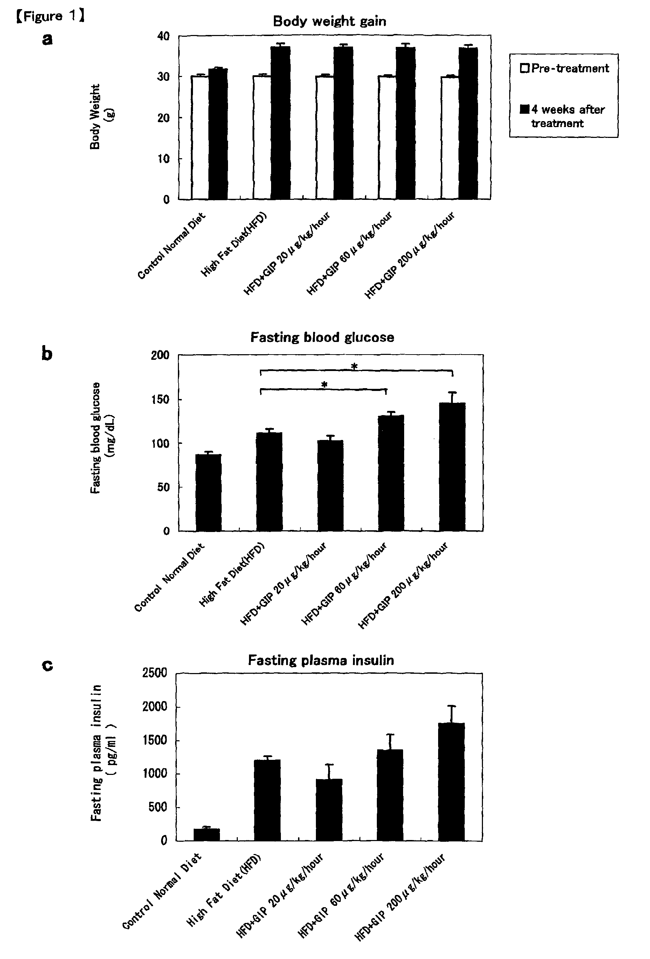

Figs. 1 respectively show the result of a high fat diet loading test in which wild type mice was given continuous administration of GIP or physiological saline as well as given control normal diet or high fat diet for four weeks, each of which is (a): a graph showing the changes in body weight over 4-week-period, (b): a graph showing the fasting blood glucose levels after 4-week treatment period, and (c): a graph showing the fasting insulin levels after 4-week treatment period. It is noted that the asterisk (*) in the figure represents p < 0.05.

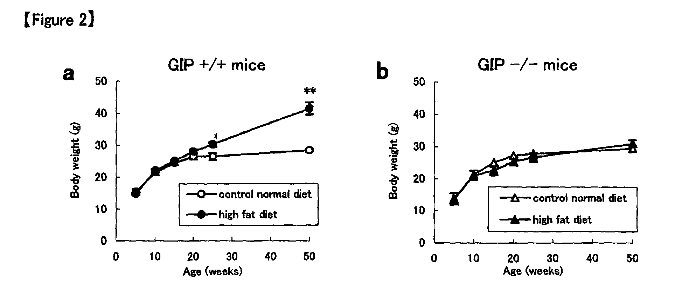

Figs. 2 respectively show the changes in body weight in wild type mice and GIP-receptor gene-deficient mice to which control normal diet or high fat diet was given, each of which is (a): a graph of the changes in body weight in wild type mice, and (b): a graph of the changes in body weight in GIP-receptor gene-deficient mice. It is noted that the asterisk (*) and double-asterisk (**) in the figure represent p < 0.05 and p < 0.01, respectively.

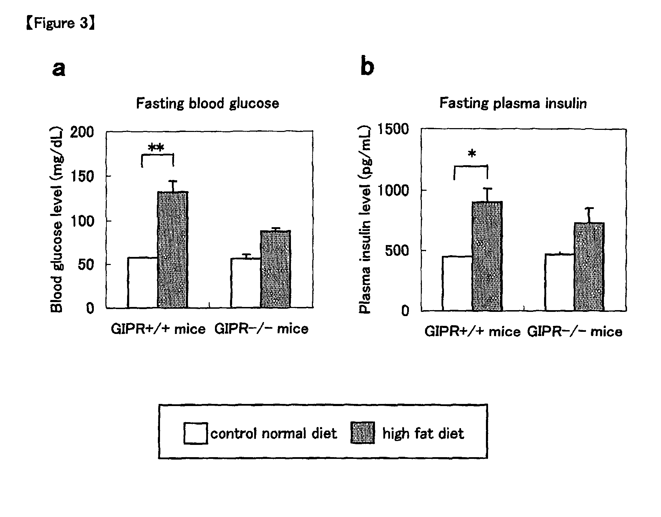

Figs. 3 respectively show the fasting blood glucose levels and the fasting insulin levels in wild type mice and GIP-receptor gene-deficient mice to which control normal diet or high fat diet was given, each of which is (a): a graph showing the fasting blood glucose levels, and (b) : a graph showing the fasting insulin levels. It is noted that the asterisk (*) and double-asterisk (**) in the figure represent p < 0.05 and p < 0.01, respectively.

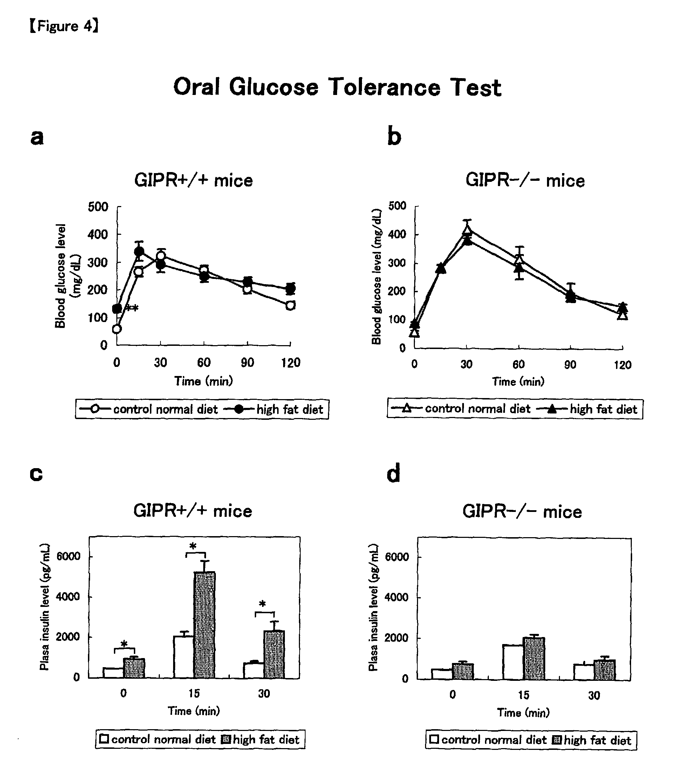

Figs. 4 respectively show the result of an oral glucose tolerance test in wild type mice and GIP-receptor gene-deficient mice to which control normal diet or high fat diet was given, each of which is (a) : a graph showing the changes in the blood glucose levels in wild type mice, (b): a graph showing the changes in the blood glucose levels in GIP-receptor deficient mice, (c): a graph showing the changes in the blood insulin levels in wild type mice, and (d): a graph showing the changes in the blood insulin levels in GIP-receptor deficient mice. It is noted that the asterisk (*) and double-asterisk (**) in the figure represent p < 0.05 and p < 0.01, respectively.

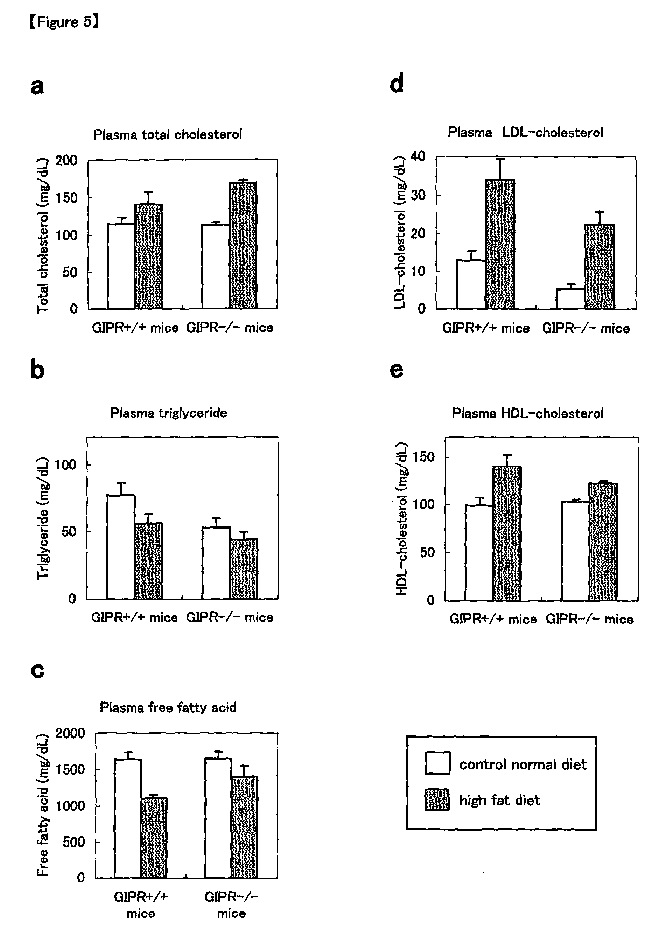

Figs. 5 respectively show the blood lipid markers in wild type mice and GIP-receptor gene-deficient mice to which control normal diet or high fat diet was given, each of which is (a): a graph showing the total cholesterol levels, (b): a graph showing the triglyceride levels, (c): a graph showing the free fatty acid levels, (d): a graph showing the LDL cholesterol levels, and (e): a graph showing the HDL cholesterol levels.

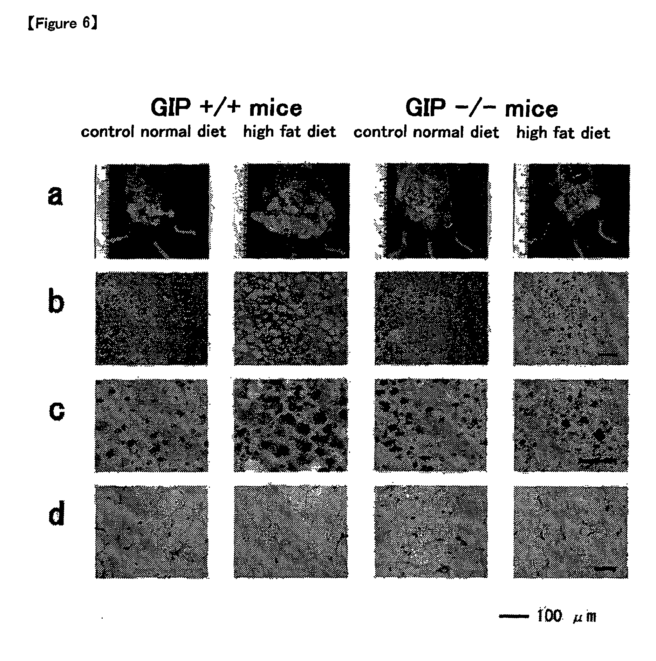

Figs. 6 respectively show the results of histological analysis in wild type mice and GIP-receptor gene-deficient mice to which control normal diet or high fat diet was given, each of which is (a): anatomic photographs of the visceral and subcutaneous adipose tissues, (b): staining photographs of the adipose tissue, (c): staining photographs of the hepatic tissues, and (d): a staining photographs of the adipocytes of the epididymal fat pad.

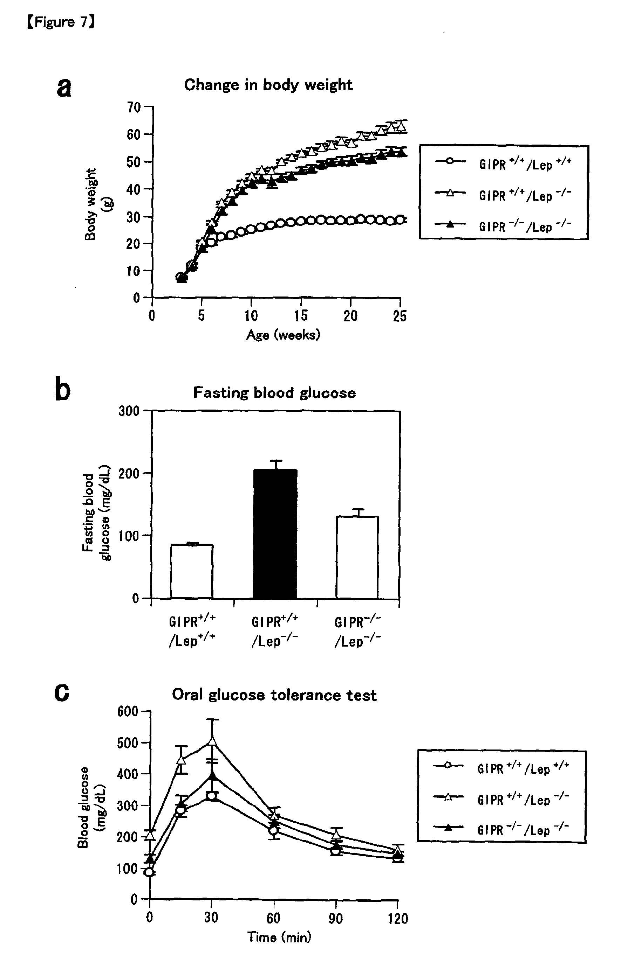

Figs. 7 respectively show the results of experiments to determine the effects of GIP-receptor defect in ob/ob mice using three strains of mice, that is, C57BL/6j-GIPR^{+/+}/Lep^{+/+}, C57BL/6j-GIPR^{+/+}/Lep^{-/-}, and C57BL/6j-GIPR^{-/-}/Lep^{-/-}, each of which is (a) : a graph showing the body weight gain, (b): a graph showing the fasting blood glucose levels, and (c): a graph showing the changes in the blood glucose levels after loading glucose.

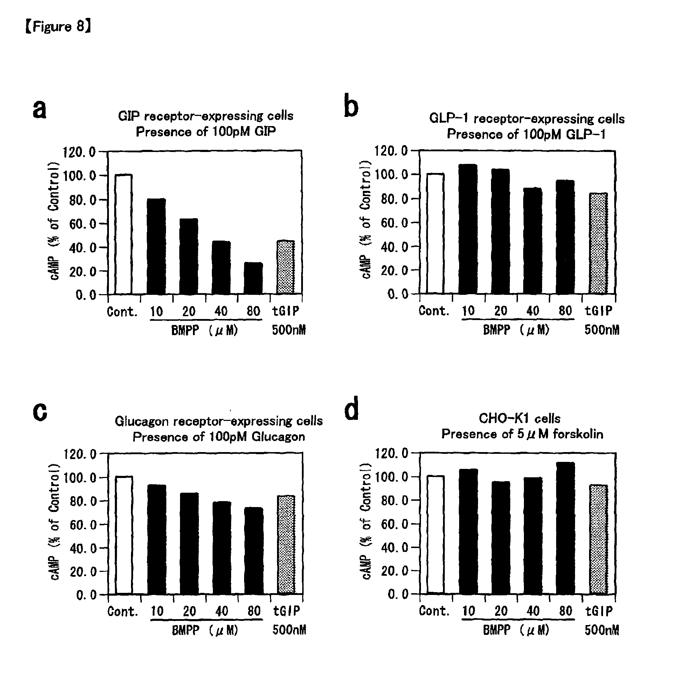

Figs. 8 respectively show the results of experiments to determine the GIP function inhibitory effect of 3-bromo-5-methyl-2-phenylpyrazolo[1,5-a]pyrimidine-7-ol (BMPP), and its specificity, each of which is (a): a graph showing the inhibitory effect of BMPP on cAMP production when the CHO cells expressing GIP-receptor were stimulated by 100 pM GIP, (b) : a graph showing the inhibitory effect of BMPP on cAMP production when the CHO cells expressing GLP-1-receptor were stimulated by 100 pM GLP-1, (c): a graph showing the inhibitory effect of BMPP on cAMP production when the CHO cells expressing glucagon-receptor were stimulated by 100 pM glucagon, and (d): a graph showing the inhibitory effect of BMPP on cAMP production when the CHO cells without transfer of receptor genes were stimulated by 5 µM forskolin.

### BEST MODE FOR CARRYING OUT THE INVENTION

The present invention relates to a prophylactic or ameliorative agent for insulin resistance and/or obesity containing a GIP function inhibitory compound as an active ingredient and is based on a completely novel concept which is different from the conventionally suggested mechanism of action of prophylactic or ameliorative agents for insulin resistance and obesity.

The present invention shows that a GIP function inhibitory compound serves as a prophylactic or ameliorative agent for insulin resistance as well as a prophylactic or ameliorative agent for obesity. However, it has been demonstrated in experiments that the prophylactic or ameliorative mechanism for insulin resistance of a GIP function inhibitory compound is not always consistent with the prophylactic or ameliorative mechanism for obesity of the same compound. In other words, it was clarified that the prophylactic or ameliorative agent for insulin resistance containing a GIP function inhibitory compound as an active ingredient, according to the present invention, dose not always exert the prophylactic or ameliorative effects for insulin resistance along with prophylaxis or amelioration of obesity. Therefore, one embodiment of prophylactic or ameliorative agent for insulin resistance containing a GIP function inhibitory compound as an active ingredient is a prophylactic or ameliorative agent for insulin resistance intended for patients with insulin resistance without obesity, and its another embodiment is a prophylactic or ameliorative agent for both insulin resistance and obesity, which is intended for obese patients and ameliorates insulin resistance in addition to obesity.

The GIP function inhibitory compound is a compound that inhibits the function of GIP at GIP gene level or GIP-receptor gene level, or at GIP itself or at GIP-receptor level, which include GIP-receptor antagonists and GIP production inhibitors. GIP-receptor antagonists include, for example, low-molecular synthetic compounds and fragmented peptides of GIP. The known fragmented peptides of GIP include GIP(6-30)-NH₂ (Regulatory Peptide, 69: 151-154, 1997) and GIP(7-30)-NH₂ (AmJPhysiol, 276:E1049-1054, 1999). Other GIP function inhibitory compounds include 3-bromo-5-methyl-2-phenylpyrazolo[1,5-a]pyrimidine-7-ol (BMPP), which has been identified in the present invention.

The prophylactic or ameliorative agent for insulin resistance and/or obesity in the present invention may be administered at various dose and in various modes based on the respective compound included, but the oral administration is preferable for low molecular compounds which can be administered orally because continuous administration is required. It is impossible to quantitate a dose generally applicable, but, as for fragmented peptides of GIP such as GIP(6-30)-NH₂ and GIP(7-30)-NH₂, it is recommended to administer subcutaneously, intramuscularly or intravenously about 0.1 to 10 mg/kg in the form of injection. As for 3-bromo-5-methyl-2-phenylpyrazolo[1,5-a]pyrimidine-7-ol (BMPP), it is considered necessary to administer about 1 to 100 mg/kg either orally in the form of powder, tablet or capsule, or subcutaneously, intramuscularly or intravenously in the form of injection. The standard manufacturing technology can apply to make preparations of them.

The present invention also relates to a screening method for a prophylactic or ameliorative agent for insulin resistance and/or obesity, characterized by selecting a GIP function inhibitory compound. The method is also based on the completely novel concept that is different from conventionally suggested screening methods of a prophylactic or ameliorative agent for insulin resistance and/or obesity.

Selection of a GIP function inhibitory compound (hereinafter, referred to as "GIP function inhibitor" in some cases) may be selection or screening of GIP-receptor antagonist, or selection of GIP production inhibitor. The compounds to be screened are preferably low molecular compounds of 600 or less molecular weight by the view of possible oral dosing.

Examples of the screening method of GIP function inhibitors (GIP-receptor antagonists and the like) can include as followings.
1. A method for screening drugs by monitoring an inhibitory activity on the production of cAMP, an intracellular transmitter of GIP, which according to the method described by Kubota et al. (Diabetes, 45: 1701-1705, 1996), uses the human GIP-receptor expressing cells where GIP cDNA is introduced into the CHO cells, and make cAMP produced by GIP in Krebs-Ringer buffer solution containing 1 mM is obutylmethylxanthine at 37°C for 30 minutes in the presence of the drug. Then cAMP is extracted with 30% trichloroacetic acid, and radioimmunoassay is used to quantitate cAMP. Alternatively, Scintillation Proximity Assay (SPA) provided by Amersham Pharmacia Biotech Co., Inc. is used to quantitate cAMP.
2. Amethod for screening drugs by monitoring an inhibitory activity on the production of cAMP, an intracellular transmitter of GIP, which according to the method described by Usdin, T. B. et al (Endocrinology, 133: 2861-2870, 1993), introduces a gene where bacteria-derived lac Z gene is linked to VIP gene-derived cAMP-dependent promoter into the human GIP-receptor expressing cells used in the above-described method 1, where GIP cDNA is introduced into the CHO cells. The resulting cells are allowed to react with GIP in Krebs-Ringer buffer solution containing 1 mM isobutylmethylxanthine at 37°C for 30 minutes in the presence of the drug. Since β-galactosidase accumulates in the cells in accordance with cAMP produced by GIP activity, this β-galactosidase activity will be used as the indicator of GIP activity.
3. A method for screening drugs by monitoring an inhibitory activity on the binding of GIP to GIP-receptor, which uses the above-described human GIP-receptor expressing cells, where the drug and ¹²⁵I-labeled GIP are added to the cells in phosphate-buffered saline (PBS (-)) solution pH7.4 containing 5.6 mM glucose and 0.5% bovine serum albumin, and the cells are incubated at 37°C for one hour, followed by washing the cells with the above-mentioned buffer and dissolving the cells in 1M sodium hydroxide, then the radioactivity in the solution is measured by γ-counter.
4. Amethod for screening drugs by monitoring an inhibitory activity on the uptake of fatty acid into the adipocytes caused by GIP, which is performed according to the method described by Beck et al. (Cellular and Molecular Biology 33(5): 555-562, 1987). Specifically, after differentiation of 3T3-L1 cells into adipocytes by using insulin, dexamethasone and isobutylmethylxanthine, the resulting adipocytes are placed in Krebs-Ringer carbonate buffer pH 7.4 containing 2% bovine albumin and 0.1% glucose, to which 0.75 mmol/L tritium-labeled palmitic acid and the drug are added, and incubated at 37°C for one hour, followed by extraction with heptane-potassium hydroxide solution andmeasurement of radioactivity of palmitic acidpresenting in the heptane layer to determine the inhibitory effect of the drug on palmitic acid uptake.
   Examples of methods for screening GIP function inhibitors, especially screening GIP production inhibitors are shown below.
5. A method according to the method described by Tseng et al. (Proc. Natl. Acad. Sci. 90: 1992-1996, 1993) where fat is perfused to the duodenum to determine the inhibition of expression of induced GIPmRNA by the drug based on the fact that GIP is secreted from the K cells in the duodenum and the jejunum in response to the stimulation by nutritional factors. Specifically, after fasting overnight, the abdomen of male SD rats (250 - 350 g) was opened under anesthesia, and a tube was inserted from the pyrorus of the stomach to perfuse 20% Intralipos (Yoshitomi Pharmaceutical Industries, Ltd.) to the duodenum for 30 to 60 minutes. At the same time, the drug is drip infused via the jugular vein. Then, the duodenum is resected, RNA is extracted by the standard method, and GIP mRNA is detected by RNA blot hybridization using GIP cDNA as a probe or by reverse transcription PCR. It is also possible to determine the inhibition of expression of induced GIP by the drug through the quantitation of GIP in the tissue or in the blood as described in a method 6 which will be described below.
6. A method to determine the effect of the drug on GIP secretion, through the quantitation of GIP produced in the cells or in conditioned medium after stimulation of the drug, using the small intestine-derived cancer cells producing a large amount of GIP which are obtained by the method described by Kieffer et al. (Am. J. Physiol, 269:E316-322, 1995). The methods available for the quantitation of GIP include high-performance liquid chromatography according to the method described by Kieffer et al. (Am. J. Physiol, 269: E316-322, 1995) and radioimmunoassay (GIP RIA kit is commercially available from Peninsula Laboratory, Inc.).
7. A method to determine the effect of the drug on GIPmRNA synthesis by quantitating GIPmRNA induced in the cells after adding the drug by the same method described in the above-described method 5, using the small intestine-derived cancer cells producing a large amount of GIP as is the case with the above-described method 6.

### EXAMPLES

### Test example 1. GIP administration and high fat diet loading test in normal mice

### (1) Methods

### 1-1) GIP administration and high fat diet loading

The animals used were 17-week-old male C57BL/6j mice. An osmotic pump was implanted under the skin of the back of each animal. Groups of animals each consisting of 5 mice were continuously given GIP 20, 60 or 200 µg/kg/hour or physiological saline as control, and also given either high fat diet or control normal diet. The energy composition of high fat diet was: lipid 45%, carbohydrate 38%, and protein 17%. The energy composition of control normal diet was: lipid 13%, carbohydrate 60%, and protein 27%. The energy contents of high fat and control normal diets were 4.77 and 3.57 kcal/g, respectively. GIP administration and high fat diet loading were continued for 4 weeks.

### 1-2) Comparison of body weight, and measurement of the blood glucose and insulin levels at fasting

The body weights were measured weekly, and their changes were compared between the groups. The blood samples were collected under fasting 4 weeks after the start of the experiment to determine the blood glucose and insulin levels.

### (2) Results

### 2-1) Body weight gain by GIP (Fig. 1a)

The body weight gain was 5.7% in 4 weeks in the normal diet group, while it was increased to 24.1% in the high fat diet group. No further increase in the body weight gain was observed in the high fat diet group given GIP.

### 2-2) Fasting blood glucose levels (Fig. 1b)

The fasting blood glucose levels increased depending on the dose of GIP in the high fat diet group with GIP administration as compared with the same diet group without GIP.

### 2-3) Fasting blood insulin levels (Fig. 1c)

The fasting blood insulin levels tended to increase depending on the dose of GIP in the high fat diet group with GIP administration as compared with the same diet group without GIP.

### (3) Consideration

Continuous administration of GIP did not affect the body weight gain in mice with high fat diet loading. However, increases in the blood glucose and insulin levels at fasting caused by high fat diet loading showed dose-dependent increases with continuous administration of GIP. This indicates that insulin resistance developed by high fat diet loading was exacerbated by GIP without accampanying progress in obesity. In other words, it was demonstrated that GIP exacerbated insulin resistance as a factor independent of obesity. Consequently, it indicates that GIP function inhibitory compound serves as a prophylactic or ameliorative agent for insulin resistance independent of prophylactic or amelioration of obesity.

### Test example 2. High fat diet loading test in GIP-receptor-deficient mice

### (1) Methods

### 1-1) High fat diet loading

The animals used were 7-week-old GIP-receptor-deficient mice and wild type mice. Groups of animals each consisting of 5 mice were given either high fat diet or control normal diet. The energy composition of high fat diet was: lipid 45%, carbohydrate 35%, and protein 20%. The energy composition of control normal diet was: lipid 13%, carbohydrate 60%, and protein 27%. The energy content was 3.57 kcal/g in both diets. Groups of animals were given the respective diet from Week 8 to Week 52, for a total of 45 weeks before oral glucose tolerance test, blood lipid profile determination and histological examination were performed to compare the groups. All groups of animals were given normal diet for the first 7 weeks. GIP-receptor-deficient mice used were male mice prepared according to the descriptionbyMiyawaki, K. etal. (Proc.Natl.Acsd. Sci. USA 96: 14843-14847, 1999).

### 1-2) Determination of blood glucose and insulin levels at fasting and glucose tolerance

Mice of all groups were fasted for 16 hours (from 18:00 to 10:00 on the following day) before determination of blood glucose and insulin levels and oral glucose loading test. For the purpose of oral glucose loading test, 2g/kg of glucose was or ally administered, and blood sample was collected from the tail vein before administration, and 15, 30, 60, 90 and 120 minutes after administration to determine the blood glucose levels. The blood glucose levels was determined by an enzyme electrode method (Sanwa Kagaku Kenkyusho Co., Ltd.), while the blood insulin levels were determined by an enzyme-labeled antibody technique (Shibayagi).

### 1-3) Determination of blood lipid profile

Mice of all groups were fasted for 16 hours (from 18:00 to 10:00 on the following day) before blood collection. Plasma was separated to determine blood lipid marker profile by an enzyme method (Kyowa Medex Co., Ltd.).

### 1-4) Histological examination of the hepatic and adipose tissues

The hepatic and adipose tissues were embedded in paraffin and 3.5-µm slices were made. After removing paraffin using xylene and ethanol, the slices were subjected to hematoxyline-eosin staining. Separately, frozen sections of the hepatic tissues were prepared and stained with oil red O.

### (2) Results

### 2-1) Body weight gain by high fat diet and control normal diet (Fig. 2)

There was no distinct difference observed in the body weight gain between GIP-receptor-deficient mice and wild type mice given with control normal diet. In wild type mice, the body weight gain was increased by 35% in those given high fat diet as compared with those given control normal diet (Fig. 2a). On the contrary, no difference in body weight gain was observed between GIP-receptor-deficient mice given high fat diet and those given control normal diet (Fig. 2b).

### 2-2) Oral glucose loading test (Fig. 3)

In wild type mice, the blood glucose and insulin levels at fasting showed a marked increase in high fat diet group as compared with control normal diet group (Figs. 3a and 3b). The group of GIP-receptor-deficient mice given high fat diet showed a distinct amelioration in hyperglycemia and hyperinsulinism at fasting observed in the group of wild type mice given high fat diet (Figs. 3a and 3b). There was no distinct difference in the peaks of blood glucose levels after glucose loading between wild type mice given high fat diet and those given control normal diet (Fig. 4a). In wild type mice given with high fat diet, the blood insulin level after glucose loading was significantly higher than that in those given control normal diet (Fig. 4c). These results indicate that insulin resistance would develop due to high fat diet loading in wild type mice. GIP-receptor-deficient mice showed a mild impaired glucose tolerance as compared with wild type mice given control normal diet, while no difference was observed in the blood glucose levels at fasting and peak as well as basal and stimulated insulin levels between GIP-receptor-deficient mice given high fat diet and those given control normal diet (Figs. 4b and 4d).

### 2-3) Blood lipid markers (Fig. 5)

The comparison of blood lipid marker profile between the groups revealed decreased triglyceride (Fig. 5b) and LDL cholesterol (Fig. 5d) levels in GIP-receptor-deficient mice as compared with wild type mice both in groups given normal and high fat diets.

### 2-4) Histological examination (Fig. 6)

Wild type mice showed a marked increase in visceral and subcutaneous adipose tissues in the high fat diet group, while no distinct difference was observed in the accumulation of visceral and subcutaneous adipose tissues between the high fat and control normal diet groups in GIP-receptor-deficient mice (Figs. 6a and 6b). Moreover, results of hematoxyline-eosin and oil red O staining revealed fatty degeneration of the hepatic tissue induced by high fat diet in wild type mice, though no such fatty degeneration of the hepatic tissue due to high fat diet loading was observed in GIP-receptor-deficientmice (Fig. 6c). In the epididymal fattypad, amarked hypertrophy of adipocytes due to high fat diet loading was observed in wild type mice, while no change was observed in adipocytes of the epididymal fatty pad in GIP-receptor-deficient mice given high fat diet (Fig. 6d).

### (3) Consideration

The body weight increase due to loading of high fat diet for 45 weeks recognized in wild typemice was not observed in GIP-receptor-deficient mice. Autopsy revealed increased visceral fat in wild type mice as compared with GIP-receptor homo-decient mice. Assuming that GIP-receptor homo-deficientmice is under the same condition wnen a GIP function inhibitory compound (e.g., GIP antagonist) is administered, this result supports that the administration of a compound inhibiting the function of GIP suppresses the fat accumulation in the adipose tissues due to high fat diet. Anyincrease in body weight was observed in neither mice fed with less fatty diet of the same calorie intake. This result indicates that the quantitative change in fat or energy taken does not determine the amount of adipose tissues, but that GIP actively regulate the amount of adipose tissues by sensitively responding of GIP to the composition of diet. Thus, GIP is considered essential for development of obesity under high fat diet loading as a thrifty gene that helps saving and accumulation of energy. Therefore, anti-obesity effect by inhibiting the function of GIP is attributable to the inhibition of promotion by GIP of uptake and accumulation of lipid in the adipose tissues. The fact that the blood lipid levels were decreased in GIP-receptor-deficient mice suggests that the lipid not taken up in the adipose tissue due to GIP-receptor deficiency does not increase the blood lipids, but that a GIP inhibitor may decrease the blood lipid levels. Results of oral glucose loading test revealed a marked increase in the blood insulin levels and hyperglycemia at fasting as well as obesity due to high fat diet in wild type mice, leading to development of insulin resistance, though no such phenomenon was observed in GIP-receptor homo-deficient mice. This fact indicates that the inhibition of GIP function improves insulin resistance. These results indicate that GIP function inhibitory compound serves as a prophylactic or ameliorative agent for not only obesity but also insulin resistance.

### Test example 3. Effect of GIP-resistance deficiency on insulin resistance and obesity in ob/ob mice.

### (1) Methods

GIP-receptor gene-deficient ob/ob mice were prepared by introducing GIP-receptor-deficient gene in ob/ob mice which suffered genetic obesity due to deficiency of gene of leptin which is an obesity-related hormone. GIP-receptor gene homo-deficient mice (G57BL/6j-GIPR^{-/-}) were mated with leptin gene hetero-deficient C57BL/6j-Lep^{+/-} mice to obtain double-hetero-deficient mice (C57BL/6j-GIPR^{+/-}/Lep^{+/-}) which lacked both GIP-receptor gene and leptin gene. Male and female animals of these C57BL/6j-GIPR^{+/-}/Lep^{+/-} were mated to obtain C57BL/6j-GIPR^{+/+}/Lep^{-/-}, C57BL/6j-GIPR^{-/-}/Lep^{-/-}, and C57BL/6j-GIPR^{+/+}/Lep^{+/+} (corresponding to wild type C57BL/6j mice). The body weight of these mice was measured weekly and their changes were compared. Four weeks after starting the experiment, mice from each group were fasted for 16 hours (from 18:00 to 10:00 on the following day) before oral glucose loading test. For the purpose of oral glucose loading test, 2 g/kg of glucose was orally administered, and blood samples were collected from the tail vein before administration, and 15, 30, 60, 90 and 120 minutes after administration to determine the blood glucose levels. The blood glucose levels were determined by an enzyme electrode method (Sanwa Kagaku Kenkyusho Co., Ltd.). The animals used in the experiment were male mice.

### (2) Results

### 2-1) Change in body weight (Fig. 7a)

The body weight of C57BL/6j-GIPR^{+/+}/Lep^{-/-} showed a marked increase as compared with that of C57BL/6j-GIPR^{+/+}/Lep^{+/+} (wild type), while the body weight gain was significantly suppressed in C57BL/6j-GIPR^{-/-}/Lep^{-/-} which lacked GIP receptor. There was no difference in food intake between C57BL/6j-GIPR^{+/+}/Lep^{-/-} and C57BL/6j-GIPR^{-/-}/Lep^{-/-} groups.

### 2-2) Fasting blood glucose levels (Fig. 7b)

C57BL/6j-GIPR^{+/+}/Lep^{-/-} showed a marked increase in the fasting blood glucose levels as compared with C57BL/6j-GIPR^{+/+}/Lep^{+/+} (wild type). A distinct amelioration in such hyperglycemia at fasting was observed in C57BL/6j-GIPR^{-/-}/Lep^{-/-}, which lacked GIP receptor.

### 2-3) Oral glucose loading test (Fig. 7c)

Oral glucose loading test revealed higher initial blood glucose levels in C57BL/6j-GIPR^{+/+}/Lep^{-/-} than C57BL/6j-GIPR^{+/+}/Lep^{+/+} (wild type), showing a distinct impaired glucose tolerance. The increase inblood glucose levels were suppressed in C57BL/6j-GIPR^{-/-}/Lep^{-/-} as compared with C57BL/6j-GIPR^{+/+}/Lep^{-/-}.

### (3) Consideration

In ob/ob mice, the function of the feeding center is disturbed because of leptin deficiency, which causes overeating, leading to development of obesity. When GIP-receptor was removed from ob/ob mice, their obesity was ameliorated, and hyperglycemia at fasting and impaired glucose tolerance were also ameliorated. These results indicate that the GIP function inhibitory compound serve as not only a prophylactic or ameliorative agent for obesity but also a prophylactic or ameliorative agent for insulin resistance.

### Test example 4. Screening of GIP function inhibitors

### (1) Methods

Screening of low molecular GIP function inhibitors was performed in accordance with the Example 1 of the screening method. Specifically, the GIP-receptor expressing CHO cells were stimulated with 100 pM GIP in the presence and absence of the test compound, and the amount of produced cAMP was measured to determine the GIP function inhibitory activity of the test compound. Inaddition, in order to confirm the receptor specificity of the test compound, the CHO cells expressing receptors of GLP-1 andglucagon which belong to glucagon-secretin family like GIP were stimulated with GLP-1 and glucagon (100 pM), respectively, and the inhibitory activity of the test compound on production of cAMP was determined. Moreover, in order to determine other effects which are unrelated to these receptors, the CHO cells without introducing any of these receptor genes were stimulated with 5 µM forskolin, and the inhibitory effect of the test compound on production of cAPM was determined. As for positive control, 500 nM GIP(7-30)-NH₂ (tGIP) was used.

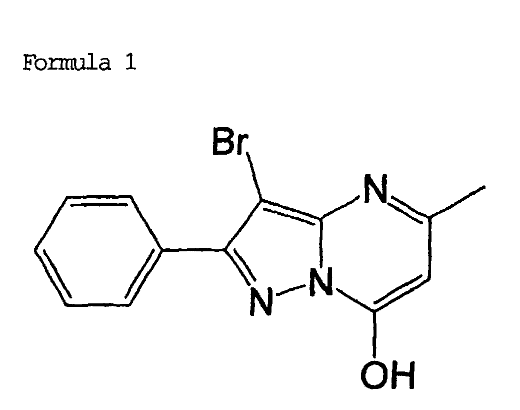

### (2) Results

The results of screening revealed that 3-bromo-5-methyl-2-phenylpyrazolo[1,5-a]pyrimidine-7-ol (BMPP; shown in Formula 1 above) purchased fromMAYBRIDGE, a library supplier, had a specific inhibitory effect on the function of GIP (Fig. 8). BMPP dose-dependently inhibited the cAMP production inducing effect of GIP in the CHO cells expressingGIP-receptor, and its EC50 was about 30 µM (Fig. 8a). BMPP slightly inhibited the action of glucagon, though its inhibitory rate was only 27% even at 80 µM (Fig. 8c), and almost no inhibitory effect on GLP-1 action was observed (Fig. 8b). Moreover, BMPP did not show any inhibitory effect on cAMP production stimulated by forskolin (Fig. 8d).

### (3) Consideration

BMPP selected as the result of screening was a test compound which may be a leading compound of low molecular GIP function inhibitors because of its high specificity for GIP-receptor in its GIP function inhibitory effect. On the other hand, it has a weak inhibitory effect on the function of glucagon, though it may be advantageous because its administration to diabetics is expected to lead the inhibitory effect on the increase in blood glucose.

The detail mechanism through which GIP induces insulin resistance is unknown at present. One possibility is that GIP affects a certain molecule inducing insulin resistance. Studies have been conducted on free fatty acid, TNF-α, etc. for molecules involved in insulin resistance. Meanwhile, the relationship between GIP and the blood free fatty acid level has been studied, and various hypotheses have been proposed. However, in the high fat diet loading test in wild type and GIP-receptor-deficient mice, no consistent relationship was observed between the presence/absence of GIP-receptor, insulin resistance and obesity, and the blood free fatty acid level under the experimental condition. There has been no specific report on the association between GIP and other factors which are considered to be involved in insulin resistance.

There are two possible mechanisms through which GIP induces obesity. The first is that GIP increases insulin secretion, and the secreted insulin increases the expression of PPARγ as well as promotes the uptake of nutrition in the adipose tissues. The second one is that GIP increases cAMP via GIP-receptors expressed in the adipocytes and directly promotes hypertrophy of adipocytes. PPARγ, which is essential for differentiation of adipocytes, is also expressed in the other organs, and its homo-deficiency is fatal. On the other hand, GIP-receptor homo-deficient mice show mild impaired glucose tolerance but no abnormality in organ formation is observed. Though GIP-receptor homo-deficient mice do not appear to be greatly different from normal mice, they show a great difference under such circumstance as high fat diet loading in that they did not show body weight gain in contrast to wild type mice whose body weight increases. Such a feature of GIP appears as the environment alters, which agrees well with the concept of thrifty genotype which appears as the environment alters and induces chronic diseases in adults. When the calorie intake is equal, the diet containing more fat is likely to cause obesity. In the absence of GIP signal, however, such a principle cannot apply, and the calorie intake determines the body weight. Thus, GIP-receptor becomes a new target of anti-obesity drugs. In other words, the GIP function inhibitory compound can be utilized for the prophylactic or treatment of obesity because of its inhibition of fat accumulation in the adipose tissues due to excessive fat intake.

As described in the section of Prior Art, it is documented in WO98/24464 that GIP antagonists has an anti-obesity effect. This cited reference reports that GIP antagonists can serve as an anti-obesity agent based on the inhibitory effect of GIP(7-30)-NH₂, a GIP antagonist, on absorption of glucose from the intestinal tract. However, it is such a logical leap to conclude that GIP antagonists serve as an anti-obesity agent based on the inhibitory effect on glucose absorption of a GIP antagonist that it can be neither recognized as a completed invention by persons skilled in the art, nor be predicted that GIP antagonists improve insulin resistance. The result of oral glucose loading test performed by us in GIP-receptor-deficient mice (Figs. 4a and 4b) revealed that the blood glucose levels after glucose administration increased in GIP-receptor-deficient mice as was the case in wild type mice, though it did not show any inhibition of glucose absorption due to GIP-receptor deficiency. Therefore, the obesity suppressing effect observed by us in GIP-receptor-deficient mice with high fat diet loading is not attributable to the inhibition of glucose absorption reported in WO98/24464. Moreover, administration of a GIP antagonist in mice is not likely to inhibit glucose absorption which leads to suppression of the blood glucose level based on the results of our experiments. In conclusion, WO98/24464 does not suggest the present invention.

## Claims

1. A prophylactic or ameliorative agent for insulin resistance, containing a GIP function inhibitory compound as an active ingredient.

2. Aprophylactic or ameliorative agent for both insulin resistance and obesity, containing a GIP function inhibitory compound as an active ingredient

3. The prophylactic or ameliorative agent for insulin resistance according to claim 1, intended for insulin resistance without obesity.

4. The prophylactic or ameliorative agent for insulin resistance according to any one of claims 1 to 3, wherein the GIP function inhibitory compound is a GIP-receptor antagonist.

5. The prophylactic or ameliorative agent for insulin resistance according to any one of claims 1 to 3, wherein the GIP function inhibitory compound is a GIP production inhibitor.

6. A screening method for a prophylactic or ameliorative agent for insulin resistance, **characterized by** selecting a GIP function inhibitory compound.

7. A screening method for a prophylactic or ameliorative agent for both insulin resistance and obesity, **characterized by** selecting a GIP function inhibitory compound.
